# EUROPEAN PATENT SPECIFICATION

(11) **EP 1 736 125 B1**
(45) Date of publication and mention of the grant of the patent: **18.03.2009**
(21) Application number: 06120792.4
(22) Date of filing: 16.01.2004
(51) Int. Cl.: A61F 5/443, A61F 5/448

(54) **A support for an ostomy bag**
Haltevorrichtung für einen Ostomiebeutel
Support pour un sac de stomie

(30) Priority: 16.01.2003 GB 0300992
(43) Date of publication of application: 27.12.2006
(62) Divisional of application: 04702742.0
(73) Proprietor: CLINIMED (Holdings) LIMITED, High Wycombe, Buckinghamshire, HP10 9QY (GB)
(72) Inventor: May, Owen c/o Clinimed (Holdings) Limited, High Wycombe Buckinghamshire HP10 9QY (GB); Smith, Rory c/o Clinimed (Holdings) Limited, High Wycombe Buckinghamshire HP10 9QY (GB)
(74) Representative: Fitchett, Stuart Paul

(56) References cited:
- EP-A1- 0 793 951
- WO-A-02/05735
- GB-A- 2 152 387
- US-A- 3 039 464
- US-A- 5 384 174
- US-A1- 2003 004 477

## Description

The present invention relates to a support for an ostomy bag, but is also applicable to wound drainage bags. For the purposes of the present specification, including the claims, all references to ostomy bags are to be interpreted to include wound-drainage bags and wound managers.

The main types of ostomy bags are colostomy bags, ileostomy bags and urostomy bags. These together with wound drainage or wound manager bags have in common that they form a seal around the stoma, or wound, through which waste material is drained from the body.

Ostomy bags comprise a receptacle for the waste having an aperture for receiving the waste. This aperture is normally surrounded by a flange of hydrocolloid adhesive. Hydrocolloid adhesive is particularly "skin friendly" comprising approximately 20% gelatine, 20% pectin and 20% carboxymethyl cellulose in an organic matrix of polyisobutylene, which comprises the remaining 40% of the hydrocolloid adhesive.

The proportions of the components of the hydrocolloid adhesive may vary, but an important property of the material is that it is breathable. This is important because a particular problem with ostomy bags arises from the extended periods for which they have to be worn by a patient, normally 24 hours a day. This commonly results in maceration where the skin cannot breath and becomes saturated.

The wear time of an ostomy bag is often limited by the seal about the flange failing, whereby waste fluids from the body may leak past the seal, requiring the wearer to replace the ostomy bag, thus replacing the seal formed with the bag. This leakage is not only unpleasant and potentially embarrassing for the wearer, but the waste is also corrosive to the skin when the skin is exposed to the waste for any significant period of time. This together with any maceration that may occur reduces the likelihood of any subsequent ostomy bag correctly sealing.

Increasing the flange size on an ostomy bag might be thought to improve the sealing ability of the flange, however it is not desired to provide an excessively large flange because this in turn will expose a larger area of skin to the possible risk of maceration and also cause problems due to the flexing of the body under the area of the flange.

Ostomy bags thus normally have a standard size of flange that is considered appropriate for the "average" wearer. However, all wearers are different and some find that the standard flange of an ostomy bag does not seal, or fails before the ostomy bag is full. This often arises where there are bony protuberances close to the stoma or where stomal hernias arise, often as a side effect of the skin being cut to form the stoma.

There are currently two products available to a wearer who suffers problems with leaking ostomy bags. The first is hydrocolloid strip, formed by extruding and rolling hydrocolloid material. The strip is cut to length by a wearer and applied in the area where the flange seal normally fails. An alternative product used by some ostomy bag wearers is sold under the trade mark "SECUPLAST". This comprises an annular disk of tape covered with an acrylic adhesive. This is placed behind and around the flange of an ostomy bag thereby extending the flange. However, a problem with this material is the acrylic adhesive is not as "skin friendly" as the hydrocolloid material and tends to increase skin trauma, which in turn causes problems with the subsequent fitting of further ostomy bags.

US-A-3 039 464 discloses a support for an ostomy bag comprising the combination of features of the preamble of claim 1.

According to the present invention there is provided a support for an ostomy bag comprising a layer of hydrocolloid shaped to fit around and extend a portion of a flange of an ostomy bag, wherein the layer of hydrocolloid is self-adhesive on one side, the said self-adhesive side of the hydrocolloid layer is covered by a release paper arranged to be removed prior to use, characterised in that the release paper is divided by two cuts into three so that it has two end sections and a middle section, and in that the hydrocolloid layer is semicircular.

Employing the present invention enables a wearer to fit a support in accordance with the present invention to that portion of the flange of an ostomy bag that is most prone to fail, when fitted to that particular wearer. The shaping of the layer of hydrocolloid, to fit around and extend the flange, ensures a large section of the circumference of the flange is extended whilst minimising the additional area of skin covered.

The invention is particularly advantageous in that it permits a standard support or range of supports to be used with one or more standard ostomy bags.

The hydrocolloid is self-adhesive on one side and covered by release paper arranged to be removed prior to use. This permits the hydrocolloid to be correctly positioned partially behind the flange and the release paper removed, such that the hydrocolloid support can be adhered to the flange in the correct position and, where the flange is provided with a similar release paper, the release paper of the flange then removed prior to mounting the ostomy bag upon the wearer.

Preferably, two hydrocolloid supports may be arranged in an opposed position about the flange, so that they extend around the complete circumference of the flange, thereby enabling the whole flange to be extended, where this is desired by a particular wearer.

The present invention will now be described, by way of example only, with reference to the accompanying drawings, in which like numerals are used throughout to indicate like parts, and of which:
Figure 1A is a plan view of a support in accordance with the present invention;
Figure 1B is a side elevation of the support of Figure 1A;
Figure 2 is a perspective view of the support of Figures 1A and 1B;
Figure 3 is a perspective view of the support of Figure 2, showing the release paper partially peeled back;
Figure 4 illustrates an ostomy bag fitted with a support in accordance with present invention; and
Figure 5 illustrates an ostomy bag fitted with two supports in accordance with present invention.

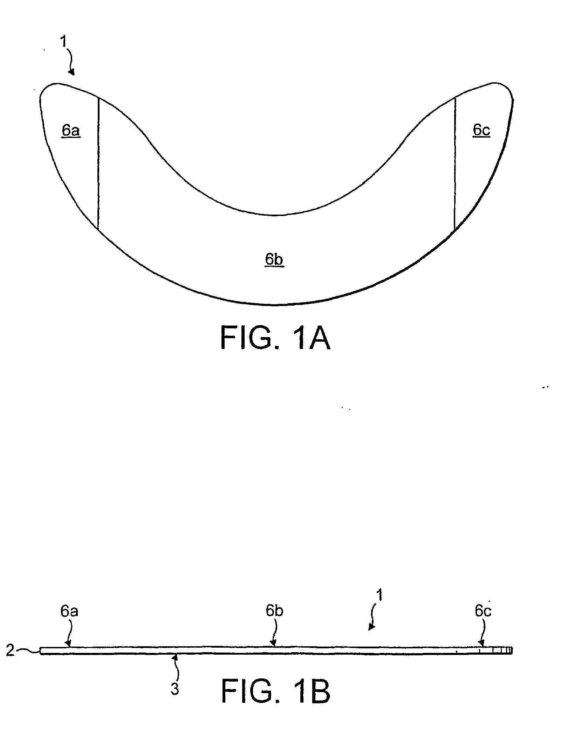

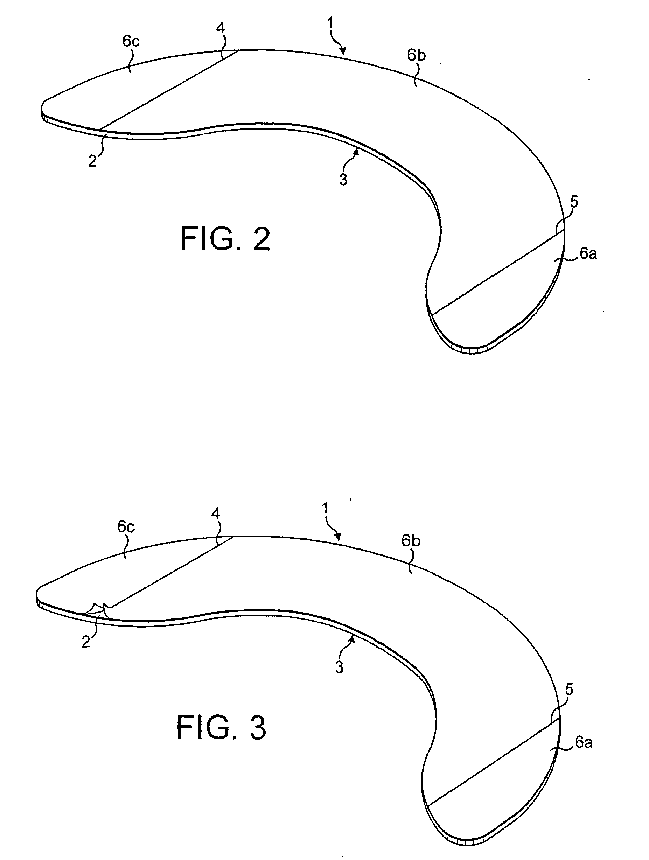

Referring to Figures 1A, 1B and 2, a support for an ostomy bag in accordance with the present invention, indicated generally as 1, comprises a layer of 0.6 mm thick Hyperflex ™ hydrocolloid, an EU40 25µm thick polyurethane film backing 3 and a release paper divided by two cuts 4 and 5 into three sections 6a, 6b and 6c, the release paper being standard sterling coated paper. The properties of the hydrocolloid material 2 and polyurethane film 3 are such that the support 1 is breathable when the release paper 6a, 6b, 6c is removed.

Figure 3 illustrates how the support 1 can be slightly flexed in the region of the slit 4 permitting the release paper to be held and peeled back. The release paper can similarly be removed starting at the slit 5.

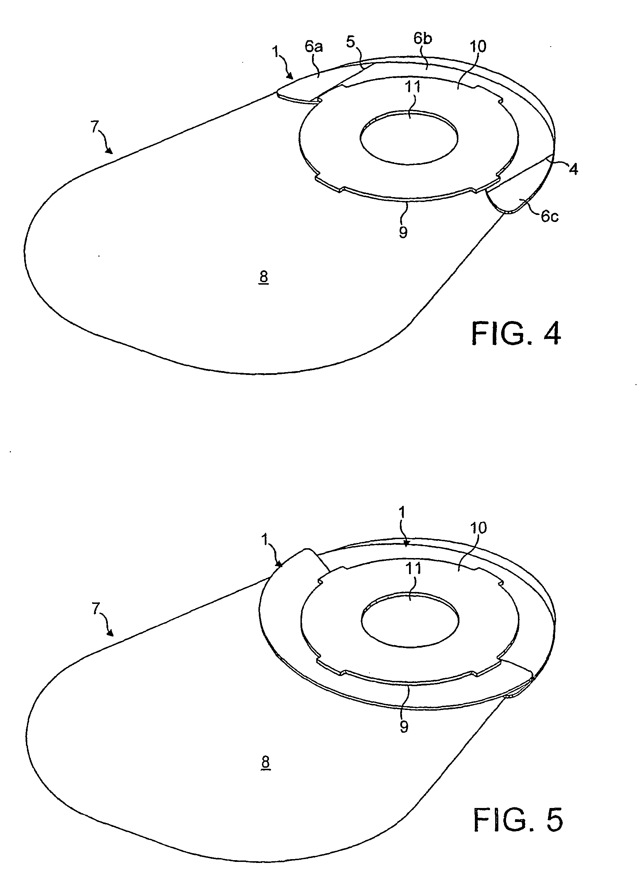

Referring to Figure 4, there is illustrated a standard ostomy bag, illustrated generally as 7, comprising a receptacle portion 8, for receiving waste, and a flange 9. The flange 9 is formed of hydrocolloid material and has a release paper on its upper surface 10. In use the release paper is removed and the flange 9 centred on a stoma or wound of the wearer, such that fluid drains from the stoma or wound into the receptacle 8, via aperture 11 in the flange 9.

The support 1 of the present invention is placed in any desired position about the flange 9, and then slid partly behind the flange with the release paper 6a, 6b, 6c uppermost, as shown. In this position, starting at one of the slits 4 or 5, the release papers are peeled from the support 1 and the support adhered to the flange 9. Then the release paper (not shown) of the flange 9 is removed from the uppermost surface 10 and the flange 9 and support 1 simultaneously adhered to the wearer.

As shown in Figure 5, a number of supports in accordance with the invention may be used on an ostomy bag, either to completely surround the flange as shown in Figure 5, to further extend the flange in a particular direction, that is to say further than it could be extended by use of a single support, or a number of supports may be built up in depth so as to account for any depression in the surface of the wearer adjacent a stoma. Although not illustrated, it is possible that supports of different sizes may also be provided.

The present invention has been illustrated by way of example only and further embodiments may be apparent within the scope of the appended claims.

## Claims

1. A support (1) for an ostomy bag (7) comprising a layer of hydrocolloid (2) shaped to fit around and extend a portion of a flange (9) of an ostomy bag, wherein the layer of hydrocolloid (2) is self-adhesive on one side, the said self-adhesive side of the hydrocolloid layer is covered by a release paper (6) arranged to be removed prior to use, **characterised in that** the release paper (6) is divided by two cuts (4, 5) into three sections so that it has two end sections (6a, 6c) and a middle section (6b), and **in that** the hydrocolloid layer (2) is semicircular.

2. A support (1) as claimed in Claim 1, and which in use can be arranged to cooperate with a similar support (1) to form a collar extending around the complete circumference of the flange (9).

## Patentansprüche

1. Haltevorrichtung (1) für einen Ostomiebeutel (7), die eine Hydrocolloidschicht (2) umfasst, die profiliert ist, um einen Teil eines Flansches (9) eines Ostomiebeutels zu passen und diesen zu erstrecken, wobei die Hydrocolloidschicht (2) auf einer Seite selbstklebend ist, die besagte selbstklebende Seite der Hydrocolloidschicht durch ein Trägerpapier (6) abgedeckt ist, die für Entfernen vor Gebrauch eingerichtet ist, **dadurch gekennzeichnet, dass** das Trägerpapier (6) durch zwei Schnitte (4, 5) in drei Abschnitte geteilt wird, sodass es zwei Seitenabschnitte (6a, 6c) und einen mittleren Abschnitt (6b) aufweist und dadurch, dass die Hydrocolloidschicht (2) halbrund ist.

2. Haltevorrichtung (1) nach Anspruch 1 und die im Gebrauch verwendet werden kann mit einer ähnlichen Haltevorrichtung (1) zu kooperieren, um einen Kragen zu bilden, der sich um den kompletten Umfang des Flansches (9) zu erstrecken.

## Revendications

1. Un support (1) pour un sac de stomie (7) comprenant une couche d'hydrocolloïde (2) formée pour s'adapter autour et prolonger une partie d'un bord (9) d'un sac de stomie, la couche d'hydrocolloïde (2) étant auto-adhésive sur un côté, ledit côté auto-adhésif de la couche hydrocolloïde étant couvert par un papier de séparation (6) conçu pour être détaché avant l'utilisation, **caractérisé en ce que** le papier de séparation (6) est divisé par deux coupes (4, 5) en trois sections de manière à posséder deux sections d'extrémité (6a, 6c) et une section intermédiaire (6b) et **en ce que** la couche d'hydrocolloïde (2) est semicirculaire.

2. Un support (1) selon le procédé de la Revendication 1, et qui en cours d'utilisation peut être conçu pour coopérer avec un support similaire (1) de manière à former un collier s'étendant autour de la circonférence complète du bord (9).
